(19) Europäisches Patentamt
European Patent Office
Office européen des brevets

(11) Numéro de publication : **0 300 861 B1**

(12) # FASCICULE DE BREVET EUROPEEN

(45) Date de publication du fascicule du brevet : **15.01.92 Bulletin 92/03**

(51) Int. Cl.$^5$ : **C07C 45/00,** C07C 45/46, C07C 47/55, C07C 47/575, C07C 47/546, C07C 47/58

(21) Numéro de dépôt : **88401660.1**

(22) Date de dépôt : **29.06.88**

(54) **Procédé de préparation d'aldéhydes aromatiques.**

(30) Priorité : **10.07.87 FR 8710180**

(43) Date de publication de la demande : **25.01.89 Bulletin 89/04**

(45) Mention de la délivrance du brevet : **15.01.92 Bulletin 92/03**

(84) Etats contractants désignés : **AT BE CH DE ES FR GB GR IT LI LU NL SE**

(56) Documents cités :
**EP-A- 0 084 742**
**AU-A- 526 054**

(73) Titulaire : **RHONE-POULENC CHIMIE**
**25, quai Paul Doumer**
**F-92408 Courbevoie Cédex (FR)**

(72) Inventeur : **Crochemore, Michel**
**3, rue des Lilas Domaine de Gilbertain**
**F-69630 Chaponost (FR)**
Inventeur : **Rochin, Christophe**
**21, rue Dussaussoy**
**F-69006 Lyon (FR)**

(74) Mandataire : **Le Pennec, Magali et al**
**RHONE-POULENC INTERSERVICES Service Brevets Chimie 25, Quai Paul Doumer**
**F-92408 Courbevoie Cédex (FR)**

EP 0 300 861 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation d'aldéhydes aromatiques. Elle concerne plus particulièrement un procédé de formylation.

Il est connu de préparer les aldéhydes aromatiques selon deux types de méthodes :

- une méthode directe qui consiste à fixer un groupe CHO sur un dérivé aromatique,
- une méthode indirecte qui consiste à oxyder un groupe déjà présent sur le dérivé aromatique.

Parmi les documents décrivant la formylation directe on peut citer le brevet US 4.588.844 qui décrit la réaction entre un dérivé aromatique et l'urotropine en milieu acide fluorhydrique.

Ce procédé utilise d'une part une matière première coûteuse : l'urotropine. Cette dernière contient 4 groupes formol potentiels et un seul d'entre eux est utilisable pour former le benzaldéhyde, en effet on doit toujours utiliser l'urotropine en quantité molaire vis à vis de l'aromatique de départ. D'autre part les rendements obtenus sur des composés tels que le fluorobenzène sont trop faibles (environ 20 %) pour envisager une exploitation industrielle.

Il est également décrit dans le brevet US 4622429 un procédé qui consiste à faire réagir un dérivé aromatique avec du monoxyde de carbone dans un milieu superacide ou en présence d'un acide de Lewis. La réaction se déroule en deux étapes et d'autre part l'emploi de monoxyde de carbone oblige l'industriel à posséder un réacteur pouvant accepter une forte pression ce qui implique un investissement coûteux.

Il est encore décrit dans le brevet CH 597149 un procédé consistant à faire réagir un formamide avec un dérivé aromatique en présence d'oxychlorure de phosphore, de phosgène ou de thiophosgène. Cette réaction ne peut être mise en oeuvre qu'avec des contraintes de sécurité importantes ce qui a toujours fait reculer l'industrie chimique.

Il est également connu d'après le brevet DE 2732227 un procédé qui consiste à faire réagir un dérivé aromatique non aminé avec l'acide cyanhydrique en milieu acide puis à effectuer une hydrolyse acide. L'utilisation de l'acide cyanhydrique impose des contraintes de sécurité équivalentes au document précédent.

Parmi les documents décrivant le deuxième mode de préparation des aldéhydes aromatiques on peut citer l'oxydation de dérivés méthylés sur le noyau aromatique (brevet GB 2165536) ou de dérivés aromatiques de l'acide glyoxalique (JP 7966639).

Ces procédés de préparation en deux étapes n'ont jamais satisfait pleinement l'industrie car le changement de réacteur est toujours nuisible à la rentabilité du procédé.

Aucun de ces procédés de formylation directe ou indirecte ne permet d'obtenir à un coût avantageux, sans normes de sécurité très dures un aldéhyde aromatique.

La présente invention a atteint cet objectif, elle a pour objet un procédé de préparation d'aldéhydes aromatiques caractérisé en ce qu'on fait réagir au sein de l'acide fluorhydrique un dérivé aromatique avec du formiate d'alkyle en présence de trifluorure de bore.

On entend par dérivé aromatique tout composé mono ou polycyclique ou hétérocyclique substitué par un ou plusieurs élément choisi parmi l'hydrogène, les halogènes, de préférence le fluor, les groupes alkyles, alkoxy, hydroxy, phénoxy, phényle.

Les dérivés aromatiques peuvent être définis par la formule (I) suivante

$$(R)_n \text{---} Ar$$

dans laquelle :

Ar représente un radical mono, ou polycyclique ou hétérocyclique,

n est un nombre entier compris entre 1 et 3

R est choisi parmi les groupes alkyle contenant 1 à 6 atomes de carbone, les groupes alkoxy contenant 1 à 6 atomes de carbone, perhalogénoalkoxy, thioalkyl contenant 1 à 6 atomes de carbone halogéno et plus particulièrement fluoro, phénoxy ou phényl éventuellement substitué.

On peut citer parmi les matières premières pouvant être mises en oeuvre dans le procédé de la présente invention, le benzène, le fluorobenzène, le chlrobenzène, l'oxyde de phényle, le gaïcol, l'anisole le biphényle.

Parmi les formiates d'alkyle utilisables on peut citer non limitativement le formiate de méthyle, d'éthyle, de n propyle, de n butyle.

L'acide fluorhydrique mis en oeuvre est de préférence anhydre. La mise en oeuvre d'acide fluorhydrique aqueux entraînerait une consommation inutile de trifluorure de bore sous forme HF, $BF_3$, $H_2O$ ($H_3O^+ BF_4^-$).

On utilise avantageusement une quantité d'acide fluorhydrique telle que le rapport molaire de l'acide fluorhydrique au dérivé aromatique soit compris entre 5 et 50.

On utilise préférentiellement une quantité de formiate d'alkyle telle que le rapport molaire de formiate d'alkyle au dérivé aromatique soit compris entre la stoechiométrie de la réaction et 2 et de préférence entre 1

et 1,5.

On préfère utiliser une quantité de trifluorure de bore telle que la pression absolue de $BF_3$ dans l'enceinte réactionelle soit comprise entre 2 et 20 bars. Une pression supérieure à 20 bars n'est pas exclue de l'invention mais n'apporte pas d'avantages particuliers.

Le procédé de l'invention est de préférence mis en oeuvre à une température comprise entre 0 et 100°C.

La durée de la réaction varie avec les matières premières mises en oeuvre ainsi qu'avec la température de réaction. Une durée de quelques heures semble convenir à la plupart des matières premières lorsqu'une température comprise entre 30 et 60°C est utilisée.

On isole facilement le produit issu de la réaction soit par distillation de l'acide fluorhydrique soit par extraction par tout solvant connu de l'homme de l'art tel que notamment : le chlorure de méthylène, l'éther isopropylique, la méthylisobutylcétone, le toluène.

Parmi les composés obtenus par le présent procédé on peut citer de manière non limitative le benzaldéhyde, le fluorobenzaldéhyde, le chlorobenzaldéhyde, le phénoxy benzaldéhyde, le méthoxy-3 hydroxy-4 benzaldéhyde, le méthoxy-4 benzaldéhyde, le méthoxy-2 benzaldéhyde, le phénylbenzaldéhyde.

Les exemples suivants sont donnés à titre indicatif et ne peuvent être considérés en aucune façon comme limitatifs de l'invention.

EXEMPLE 1 : formylation du fluorobenzène par le formiate de méthyle en milieu HF : $BF_3$.

Un solution de fluorobenzène (0,025 mole, 2,4 g) dans du formiate de méthyle (0,033 mole, 2 g) est introduite à −5°C dans 20 g d'HF anhydre. Le mélange est mis sous pression de 10 bar de $BF_3$, puis porté 6 heures à 60°C.

Après refroidissement à 0°C, le mélange réactionnel est soutiré sur 80 g de glace, extrait avec 100 ml d'éther isopropylique, neutralisé avec une solution saturée de bicarbonate de potassium.

La phase organique est séparée et séchée sur KF.

Les rendements sont déterminés par chromatographie phase gazeuse.

Plusieurs substrats aromatiques ont été soumis à cette réaction de formylation par HCOOMe dans le rapport suivant 0,025 mole de substrat par 0,033 mole de formiate de méthyle. Le tableau qui suit décrit les conditions mises en oeuvre et rassemble les résultats obtenus :

— taux de conversion du substrat,
— nature de l'aldéhyde formé,
— sélectivité en aldéhyde.

| Substrats | Pression BF3 (bar) | T (°C) | t (heures) | TT (%) | Produits | RT (%) |
|---|---|---|---|---|---|---|
| 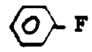 | 10 | 60 | 6 | 95 | 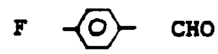 | 85 |
| | 2,5 | 40 | 4 | 55 | | 92 |
| | 5 | 50 | 5 | 80 | | 63 |
| 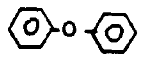 | 10 | 50 | 5 | 85 | 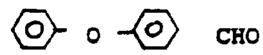 | 63 |
| | 10 | 30 | 5 | 20 | | 63 |
| 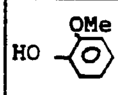 | 5 | 50 | 5 | 90 | 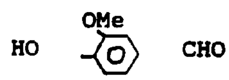 | 45 |
| 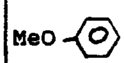 | 10 | 50 | 4 | 25 | 45 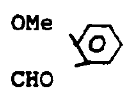 55 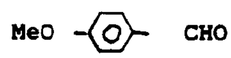 | 95 |
| | 5 | 50 | 5 | 25 | 58 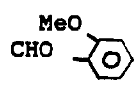 42 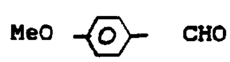 | 95 |
| 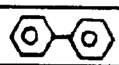 | 10 | 60 | 6 | 55 | 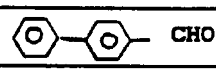 | 95 |

**Tableau I** : formylation par le formiate de méthyle en milieu HF : $BF_3$

EXEMPLE 2

On reproduit l'exemple 1 avec le fluorobenzène en remplaçant le formiate de méthyle par le formiate d'éthyle.

Après 6 heures de réaction à 60°C on obtient 85% de perafluorobenzaldéhyde.

$$(R)_n — Ar (I)$$

## Revendications

1. Procédé de préparation d'aldéhydes aromatiques caractérisé en ce qu'on met en présence dans l'acide

fluorhydrique liquide un dérivé aromatique de formule générale (I).

$$(R)_n — Ar \quad (I)$$

dans laquelle :

— Ar représente un radical mono, polycyclique ou hétérocyclique

— n est un nombre entier compris entre 1 et 3

— R est choisi parmi les groupes alkyles contenant 1 à 6 atomes de carbone, les groupes alkoxy contenant 1 à 6 atomes de carbone, perhalogénoalkoxy, thioalkyl contenant 1 à 6 atomes de carbone halogéno, et plus particulièrement fluoro, phénoxy ou phényl éventuellement substitué avec un formiate d'alkyle en présence de trifluorure de bore.

2. Procédé selon la revendication 1 caractérisé en ce que le composé de formule I est choisi parmi le fluorobenzène, l'oxyde de biphényle et le biphényle.

3. Procédé selon la revendication 1 caractérisé en ce que le formiate d'alkyle est le formiate de méthyle.

4. Procédé selon la revendication 1 caractérisé en ce que le rapport molaire de l'acide fluorhydrique au dérivé aromatique est de préférence compris entre 5 et 50.

5. Procédé selon la revendication 1 caractérisé en ce que le rapport molaire du formiate d'alkyle au dérivé aromatique est compris entre 1 et 1,5.

6. Procédé selon la revendication 1 caractérisé en ce que la pression du trifluorure de bore est de préférence comprise entre 1 et 20 bars.

7. Procédé selon la revendication 1 caractérisé en ce que la température de réaction est comprise entre 0 et 100°C et de préférence entre 30° et 60°C.

## Patentansprüche

1. Verfahren zur Herstellung von aromatischen Aldehyden, dadurch gekennzeichnet, daß man in flüssiger Fluorwasserstoffsäure eine aromatische Verbindung der allgemeinen Formel (I)

$$(R)_n — Ar \quad (I)$$

in der

— Ar einen mono-, poly- oder heterocyclischen Rest bedeutet,

— n eine ganze Zahl zwischen 1 und 3 ist und

— R aus den 1 bis 6 Kohlenstoffatome enthaltenden Alkyl-, 1 bis 6 Kohlenstoffatome enthaltenden Alkoxy-, Perhalogenalkoxy-, 1 bis 6 Kohlenstoffatome enthaltenden Thioalkylgruppen, Halogenresten, insbesondere Fluor, und gegebenenfalls substituierten Phenoxy- oder Phenylgruppen ausgewählt ist, mit einem Alkylformiat in Gegenwart von Bortrifluorid in Berührung bringt.

2. Verfahren nach Anspruch 1, dadurch gekennzeichnet, daß die Verbindung der Formel I aus Fluorbenzol, Biphenyloxid und Biphenyl ausgewählt ist.

3. Verfahren nach Anspruch 1, dadurch gekennzeichnet, daß das Alkylformiat Methylformiat ist.

4. Verfahren nach Anspruch 1, dadurch gekennzeichnet, daß das Molverhältnis von Fluorwasserstoffsäure zur aromatischen Verbindung vorzugsweise zwischen 5 und 50 beträgt.

5. Verfahren nach Anspruch 1, dadurch gekennzeichnet, daß das Molverhältnis von Alkylformiat zur aromatischen Verbindung zwischen 1 und 1,5 beträgt.

6. Verfahren nach Anspruch 1, dadurch gekennzeichnet, daß der Bortrifluoriddruck vorzugsweise zwischen 1 und 20 bar liegt.

7. Verfahren nach Anspruch 1, dadurch gekennzeichnet, daß die Reaktionstemperatur zwischen 0 und 100°C und vorzugsweise zwischen 30 und 60°C liegt.

## Claims

1. Process for the preparation of aromatic aldehydes, in which an aromatic derivative of general formula (I)

$$(R)_n — Ar \quad (I)$$

in which :

— Ar denotes a mono, polycyclic or heterocyclic radical

— n is an integer between 1 and 3, and

— R is chosen from alkyl groups containing 1 to 6 carbon atoms, alkoxy groups containing 1 to 6 carbon atoms, perhaloalkoxy or thioalkyl groups containing 1 to 6 carbon atoms, and halo and more particularly fluoro, phenoxy or optionally substituted phenyl groups, is brought into contact with an alkyl formate in liquid hydrofluoric acid in the presence of boron trifluoride.

2. Process according to Claim 1, characterised in that the compound of formula I is chosen from fluorobenzene, diphenyl ether and biphenyl.

3. Process according to Claim 1, characterised in that the alkyl formate is methyl formate.

4. Process according to Claim 1, characterised in that the molar ratio of hydrofluoric acid to the aromatic derivative is preferably between 5 and 50.

5. Process according to Claim 1, characterised in that the molar ratio of the alkyl formate to the aromatic derivative is between 1 and 1.5.

6. Process according to Claim 1, characterised in that the boron trifluoride pressure is preferably between 1 and 20 bars.

7. Process according to Claim 1, characterised in that the reaction temperature is between 0 and 100°C and preferably between 30° and 60°C.